# EUROPEAN PATENT SPECIFICATION

(11) **EP 4 137 174 B1**
(45) Date of publication and mention of the grant of the patent: **31.07.2024**
(21) Application number: 20932418.5
(22) Date of filing: 03.11.2020
(51) Int. Cl.: A61M 5/142, A61M 5/14, A61M 5/145, A61M 39/28

(54) **INFUSION BOX OF INFUSION APPARATUS, INFUSION APPARATUS, AND INFUSION PUMP**
INFUSIONSBOX EINER INFUSIONSVORRICHTUNG, INFUSIONSVORRICHTUNG UND INFUSIONSPUMPE
BOÎTE DE PERFUSION D'APPAREIL DE PERFUSION, APPAREIL DE PERFUSION ET POMPE À PERFUSION

(30) Priority: 24.04.2020 CN 202010330062; 24.04.2020 CN 202010330276
(43) Date of publication of application: 22.02.2023
(73) Proprietor: Changsha Magill Medical Technology Co., Ltd., Changsha, Hunan 410205 (CN)
(72) Inventor: LI, Daqing, Changsha, Hunan 410205 (CN); XU, Xiong, Changsha, Hunan 410205 (CN); LIU, Zhonghui, Changsha, Hunan 410205 (CN); FENG, Tieqi, Changsha, Hunan 410205 (CN)
(74) Representative: Brevalex
(86) International application number: PCT/CN2020/126286
(87) International publication number: WO 2021/212805

(56) References cited:
- WO-A1-95/27852
- WO-A1-95/31233
- CN-A- 102 292 118
- CN-U- 203 447 573
- CN-U- 205 252 208
- US-A- 5 415 528
- US-A- 5 568 912
- US-B2- 8 974 416

## Description

### TECHNICAL FIELD

The disclosure relates to technologies of medical devices, and in particular to an infusion cassette of an infusion set, an infusion set and an infusion pump.

### BACKGROUND

In clinical practice, liquids generally need to be infused into the body of a patient in a very precise way. In this case, it is often difficult for gravity infusion devices to meet the requirements. Instead, the dose and the rate of liquid are adjusted by an infusion pump for accurate transfusion. Therefore, in recent years, infusion pumps have been used widely, and various types of infusion pumps have also appeared, such as infusion pumps for average fluid infusion, ambulatory patients controlled analgesia pumps, infusion pumps for blood transfusion and chemotherapy, or the like.

In related art of the infusion pump, the pump body squeezes the tube of infusion set through a peristaltic movement thereof to control the flow of the liquid in the infusion set. A type of infusion pump rigidly squeezes the PVC infusion tube of the infusion set through the peristaltic fingers according to a timing sequence to control the flow of the liquid in the infusion set. The PVC infusion tube often has a risk of damage and leakage caused by repeated squeezing by the peristaltic fingers, and the overall dimension of the infusion pump is relatively large and the weight thereof is large. In addition, there is an infusion pump, which squeezes the flexible silicone tube arranged in the infusion cassette of the infusion set through the peristaltic fingers according to a timing sequence to control the flow of the liquid in the infusion set. The two ends of the silicone tube are fixed, and other parts of the silicone tube between the two ends are arranged horizontally along an extrusion plate and are in a relatively free state. Generally speaking, the peristalsis of the peristaltic fingers of the pump body may drive the silicone tube into a random displacement and swing, which will adversely affect the accuracy of the transfusion to a certain extent. Furthermore, when the silicone tube is assembled with the extrusion plate, the variation of the length of the tube and the variation of the gap between the tube and the extrusion plate may bring about the variation of the accuracy of the transfusion. In addition, for those types of infusion pumps, higher requirements are put forward for the fit clearance between the peristaltic fingers of the pump body and the fixing hole of the peristaltic finger frame. If the clearance is larger, the peristaltic fingers may swing and fail to squeeze the predetermined position of the silicone tube accurately. If the clearance is smaller, the friction between the peristaltic fingers and the fixing hole of the peristaltic finger frame will easily occur, which will affect the life span of the pump head and reduces the torque of the peristaltic fingers.

Generally speaking, the infusion tube of the infusion set communicates with the liquid container. When the infusion cassette or the infusion tube and the pump body are assembled together, the peristaltic fingers of the pump head may squeeze the PVC infusion tube or the silicone tube in the infusion cassette to control the free flow of the liquid in the infusion set. However, when the PVC infusion tube or infusion cassette is disengaged from the pump body, if there is no matching device for stopping the liquid, the liquid in the infusion tube flows out freely under the action of gravity. This kind of free flow is in an uncontrolled state, which causes great risks and inconvenience in some clinical application scenarios.

US5568912A discloses a sliding flow controller for controlling flow through a pumping segment used in a fluid delivery system. The flow controller includes a slider that travels along a channel defined by an elastomeric membrane and a variable size groove formed in a rigid component. The slider includes a projecting ball that presses the membrane against the groove. At one end the groove has a maximum cross-sectional area for a maximum flow position and at another end, the groove has no cross-sectional area for a flow stop position. The slider and the pumping segment have respective click stops to impart sensory feedback to the operator when manually operated. A ramped thumb indentation is provided on the exterior of the slider to facilitate sliding movement.

WO95/31233A1 discloses a simple disposable infusion pumping cassette provided for use with a drug infusion pump. The cassette includes internal means for mounting an elastomeric member between a face member and a base member and in fluidtight engagement within the cassette. The cassette includes a pumping chamber and a proximal and a distal pressure monitoring chamber adjacent thereto. A flow stop mounted on the cassette is operative to prevent fluid flow through the cassette in a first mode and to monitor fluid pressure in the cassette in a second mode.

### SUMMARY

In view of this, the embodiments of the disclosure are expected to provide an infusion cassette of an infusion set, an infusion set and an infusion pump, which may have high accuracy and easy manufacturing.

To achieve the above purpose, according to a first aspect of the disclosure, there is provided an infusion cassette of an infusion set, the infusion cassette being provided with a flow channel, and including:
a rigid assembly including a plurality of components stacked onto one another, where at least one of the plurality of components is provided with a groove;
an elastic membrane arranged between two adjacent components of the plurality of components to cover the groove in a sealing manner, where the elastic membrane and the groove together form at least a portion of the flow channel; and
a locking mechanism, where the locking mechanism includes a mounting frame and a liquid stop plug fixedly arranged on the mounting frame, the liquid stop plug is configured to press the elastic membrane to close the flow channel, the locking mechanism closes or opens the flow channel by means of the liquid stop plug, where the mounting frame is movably connected with a main body frame to allow the locking mechanism to switch between a closed state in which the flow channel is closed and an open state in which the flow channel is opened.

The plurality of components includes a first component, a second component and a third component. The groove includes a first groove and a second groove. The elastic membrane includes a first elastic membrane and a second elastic membrane. The second component is arranged between the first component and the third component. The first groove is formed on a side of the second component facing toward the first component, and the first elastic membrane is arranged between the first component and the second component to cover the first groove in a sealing manner. The second groove is formed on a side of the second component away from the first component. The second groove communicates with the first groove and is located downstream of the first groove. The second elastic membrane is arranged between the second component and the third component to cover the second groove in a sealing manner. The liquid stop plug is arranged on a side of the third component away from the second component, and the liquid stop plug is configured to press the second elastic membrane to close the flow channel.

In some embodiments, the infusion cassette further includes an elastic member configured to exert a force on the mounting frame to allow the locking mechanism to keep in the closed state in which the flow channel is closed.

In some embodiments, the open state includes a first open state, and the locking mechanism keeps in the first open state under an action of an external force. When the external force is absent or an elastic force of the elastic member overcomes an acting force of the external force on the locking mechanism, the elastic member drives the locking mechanism to automatically switch from the first open state to the closed state.

In some embodiments, the open state includes a second open state, and the locking mechanism includes a latch structure arranged on the mounting frame. The latch structure is configured to be selectively lock fitted with the rigid assembly to lock the locking mechanism in the second open state.

In some embodiments, the rigid assembly is provided with two through holes penetrating through the rigid assembly along a thickness direction of the rigid assembly. The flow channel is located between the two through holes. The mounting frame includes a cross bar, a connecting bar and slidable bars. Each of the slidable bars is arranged in respective one of the two through holes. The connecting bar is arranged on one side of the rigid assembly along the thickness direction, and the cross bar is arranged on another side of the rigid assembly opposite to said one side along the thickness direction. A first end of each of the two slidable bars is connected to the connecting bar, and a second end of each of the two slidable bars is connected to the cross bar. The liquid stop plug is arranged on the cross bar.

In some embodiments, the latch structure protrudes from surfaces of the two slidable bars. A chute is formed on a wall surface of each of corresponding through holes. The first component is provided with a stopper protruding towards the through holes. When the two slidable bars slide to a position where an interference between the latch structure and the stopper is absent, the locking mechanism is capable to swing along a length direction of the infusion cassette, and the latch structure slides into a rim of the chute and abuts against a side of the stopper facing toward the second component, to allow the locking mechanism to be locked in the second open state.

According to a second aspect of the disclosure, there is provided an infusion set, including: a plurality of infusion tubes and any of the infusion cassettes described above. The infusion cassette is provided with a liquid inlet and a liquid outlet. One infusion tube of the plurality of infusion tubes is connected to the liquid inlet, and another infusion tube of the plurality of infusion tubes is connected to the liquid outlet. The plurality of infusion tubes communicate with the flow channel.

According to a third aspect of the disclosure, there is provided an infusion pump, including: a pump body and an infusion set. The infusion set includes a plurality of infusion tubes and the infusion cassette according to the first aspect of the disclosure described above. The infusion cassette is provided with a liquid inlet and a liquid outlet. One infusion tube of the plurality of infusion tubes is connected to the liquid inlet, and another infusion tube of the plurality of infusion tubes is connected to the liquid outlet. The plurality of infusion tubes communicate with the flow channel. The pump body and the infusion cassette detachably cooperate with each other.

The first elastic membrane and the first groove together form at least a portion of the flow channel. The first groove includes a pump chamber, a first actuating chamber located upstream of the pump chamber, and a second actuating chamber located downstream of the pump chamber. The pump chamber, the first actuating chamber and the second actuating chamber are arranged in a straight line.

The pump body is arranged on a side of the first component away from the second component. The pump body includes a pump chassis, a camshaft rotatably arranged on the pump chassis, a plunger assembly press-fitted with the pump chamber, a first valve actuator assembly press-fitted with the first actuating chamber, a second valve actuator assembly press-fitted with the second actuating chamber, and a power unit configured to drive the camshaft into rotation. Each of an end of the plunger assembly, an end of the first valve actuator assembly and an end of the second valve actuator assembly abuts against a rotating surface of the camshaft and is slidable on the rotating surface of the camshaft. The camshaft drives the plunger assembly, the first valve actuator assembly, and the second valve actuator assembly to reciprocate in timing sequence to press the first elastic membrane during rotation, to generate a directional flow of liquid in the flow channel.

In some embodiments, the liquid inlet is arranged at a first end of the infusion cassette along a length direction of the infusion cassette. The liquid outlet is arranged at a second end of the infusion cassette along the length direction. The flow channel, the liquid inlet and the liquid outlet are arranged along a straight line in a projection on a plane perpendicular to a thickness direction of the infusion cassette.

In some embodiments, the open state includes a first open state. During assembling of the infusion cassette and the pump body, the pump chassis forces the mounting frame to move toward of the third component, to drive the locking mechanism to switch from the closed state to the first open state and keep the locking mechanism in the first open state.

In some embodiments, the infusion cassette includes an elastic member configured to exert a force on the mounting frame to allow the locking mechanism to keep in the closed state in which the flow channel is closed.

In some embodiments, the open state includes a second open state, and the locking mechanism includes a latch structure arranged on the mounting frame. At least one of the first component, the second component and the third component is lock fitted with the latch structure to allow the locking mechanism to switch to the second open state and keep the locking mechanism in the second open state.

In some embodiments, a part of the mounting frame protrudes from a side of the first component facing toward the pump body. A protruding post configured to push the mounting frame is formed on a side of the pump chassis facing toward the infusion cassette, and an inclined surface is formed on an end of the protruding post facing toward the infusion cassette. During the assembling of the infusion cassette and the pump body, the inclined surface pushes the mounting frame to move toward the third component, to fix the locking mechanism in the first open state.

In some embodiments, the camshaft is rotatable in a forward direction and a reverse direction. When the camshaft rotates in the forward direction, the camshaft drives the plunger assembly, the first valve actuator assembly and the second valve actuator assembly to reciprocate in a forward timing sequence, to drive the liquid in the flow channel to flow in the forward direction. When the camshaft rotates in the reverse direction, the camshaft drives the plunger assembly, the first valve actuator assembly and the second valve actuator assembly to reciprocate in reverse timing sequence, to drive the liquid in the flow channel to flow in the reverse direction.

In the infusion cassette according to the embodiments of the disclosure, each of the plurality of components of the rigid assembly is a rigid injection molding part, and the elastic membrane is a flexible injection molding part. The injection molding part has high processing accuracy, simple assembly, small assembly error and good product consistency. In addition, since the elastic membrane is arranged between two adjacent components of the plurality of components, and when the elastic membrane is pressed, the possibility of unexpected displacement and swing of the elastic membrane is very small. The locking mechanism selectively closes the flow channel, which can prevent excessive infusion or environmental hazards by the liquid medicine due to the free flow of the liquid in the infusion cassette. In addition, the elastic membrane itself has good extrusion resistance, so that the risk of leakage caused by the damage of the elastic membrane due to repeated pressing is very low.

### BRIEF DESCRIPTION OF THE DRAWINGS

FIG. 1 is a schematic diagram of an infusion cassette according to an embodiment of the disclosure;
FIG. 2 is an explosion diagram of the structure shown in FIG. 1;
FIG. 3 is a schematic diagram of a second component shown in FIG. 1;
FIG. 4 is a schematic diagram of a locking mechanism shown in FIG. 1;
FIG. 5 is a cross-sectional view of the infusion cassette shown in FIG. 1;
FIG. 6 is a schematic diagram of an infusion pump according to an embodiment of the disclosure, where a pump body and an infusion cassette are in a disengaged state;
FIG. 7 is a schematic diagram of the assembled infusion pump shown in FIG. 6, in which part of the structure is shown in sectional view.

### DETAILED DESCRIPTION

It should be noted that the embodiments in the disclosure may be combined with each other and the technical features in the embodiments may be combined with each other without conflict. The detailed description in the specific embodiments should be understood as an explanation of the purpose of the disclosure, and should not be regarded as an improper limitation of the disclosure.

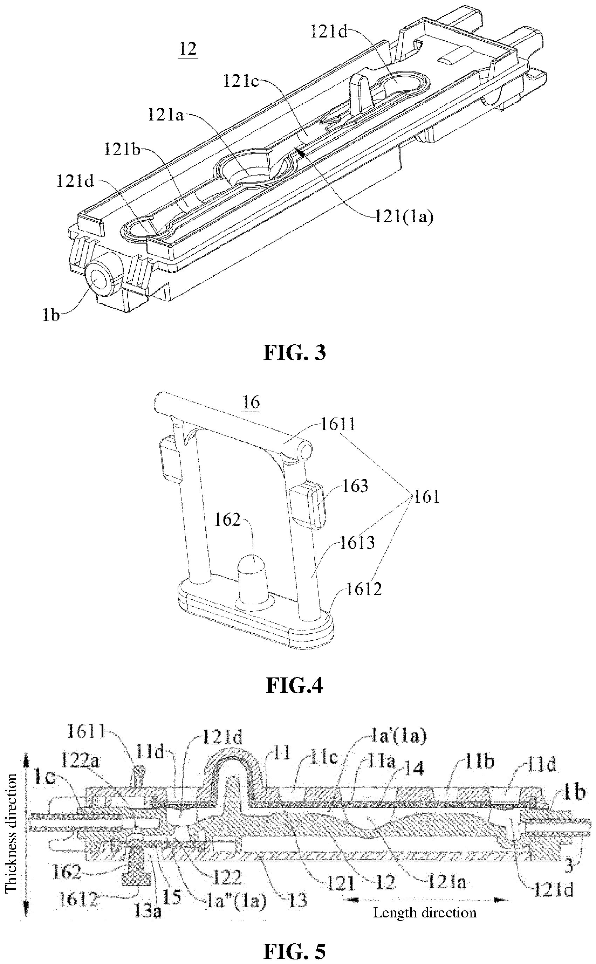

In the description of embodiments of the disclosure, orientation or positional relationship in "thickness direction" and "length direction" is based on the orientation or positional relationship shown in FIG. 5. It is to be understood that these orientation terms are only for the convenience of describing the disclosure and simplifying the description, rather than indicating or implying that the device or element in question shall have a particular orientation, and shall be constructed and operate in a particular orientation. Therefore, these orientation terms should not be construed as a limitation of the disclosure.

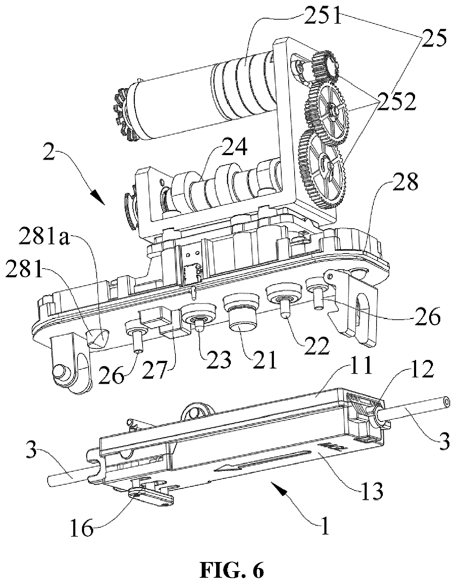

The embodiment of the disclosure provides an infusion pump. With reference to FIG. 6, the infusion pump includes a pump body 2 and an infusion set.

The infusion set includes an infusion cassette 1 and a plurality of infusion tubes 3 connected with the infusion cassette 1. It will be appreciated that in some embodiments, the infusion set may further include other accessories arranged on the plurality of infusion tubes 3. The specific types of the accessories may be determined according to the actual use condition, and will not be described herein in detail.

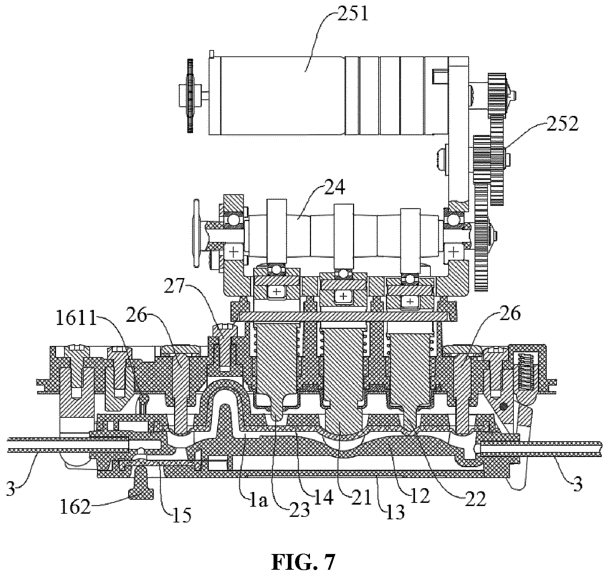

The pump body 2 and the infusion cassette 1 detachably cooperate with each other. Specifically, with reference to FIG. 7, when an infusion pump needs to be used, the infusion cassette 1 may be in snap-fit with the pump body 2. With reference to FIG. 6, at the end of the use, the pump body 2 and the infusion cassette 1 can be disengaged from each other. The infusion cassette 1 is disposable consumable, and the pump body 2 is a reusable device.

With reference to FIG. 5, the infusion cassette 1 is provided with a liquid inlet 1b, a liquid outlet 1c, and a flow channel 1a communicating with the liquid inlet 1b and the liquid outlet 1c. One infusion tube 3of the plurality of infusion tubes 3 is provided with an end connected to the liquid inlet 1b and another infusion tube 3 of the plurality of infusion tubes 3 is provided with an end connected to the liquid outlet 1c, and the plurality of infusion tubes 3 communicate with the flow channel 1a.

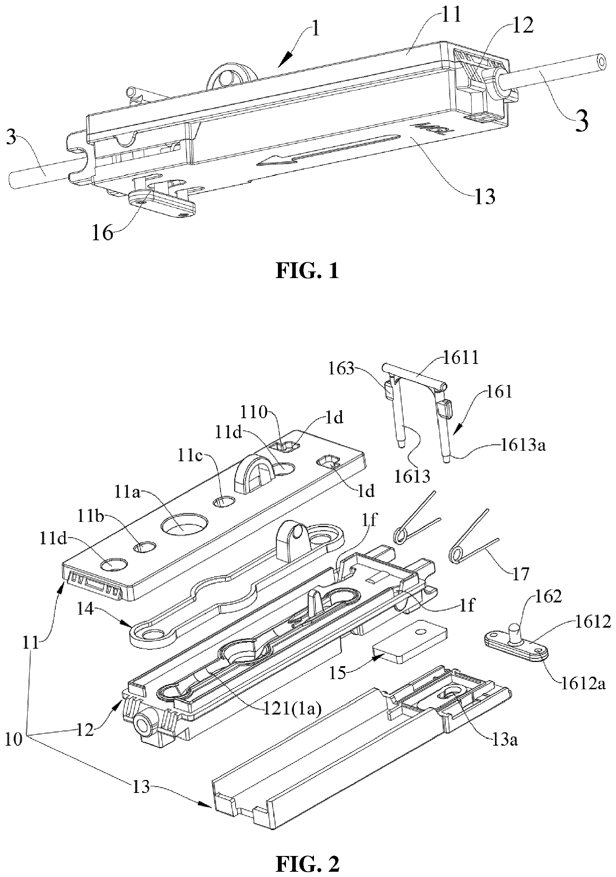

With reference to FIG. 2, the infusion cassette 1 includes a rigid assembly 10 and an elastic membrane. The rigid assembly 10 includes a plurality of components stacked onto one another, and at least one of the plurality of components is provided with a groove. The elastic membrane is arranged between two adjacent components of the plurality of components to cover the groove in a sealing manner, and the elastic membrane and the groove together form at least a portion of the flow channel.

In the embodiment of the disclosure, based on the structural characteristics of the flow channel formed by the elastic membrane and the groove, the infusion cassette 1 further includes a locking mechanism 16 to prevent excessive transfusion or environmental hazards caused by uncontrolled free flow of a liquid in the infusion set.

With reference to FIG. 2 and FIG. 4, the locking mechanism 16 includes a mounting frame 161, and a liquid stop plug 162 fixedly arranged on the mounting frame 161. That is, the mounting frame 161 and the liquid stop plug 162 move synchronously. The liquid stop plug 162 is configured to press the elastic membrane to close the flow channel 1a, and the locking mechanism 16 closes or opens the flow channel 1a by means of the liquid stop plug 162. The mounting frame 161 is movably connected with the rigid assembly 10 to allow the locking mechanism 16 to switch between a closed state in which the flow channel 1a is closed and an open state in which the flow channel 1a is opened.

It should be noted that the locking mechanism 16 performs the function of closing or opening the flow channel 1a through the liquid stop plug 162, and the position of the liquid stop plug 162 may be changed by the movement of the mounting frame 161 relative to the rigid assembly 10 of the main architecture.

With reference to FIG. 3, in an embodiment, the groove 121 includes a pump chamber 121a, a first actuating chamber 121b located upstream of the pump chamber 121a, and a second actuating chamber 121c located downstream of the pump chamber 121a.

In an embodiment, the pump body 2 is arranged on a side of a first component 11 away from a second component 12. With reference to FIG. 6, the pump body 2 includes a pump chassis 28, a camshaft 24 rotatably arranged on the pump chassis 28, a plunger assembly 21 press-fitted with the pump chamber 121a, a first valve actuator assembly 22 press-fitted with the first actuating chamber 121b, a second valve actuator assembly 23 press-fitted with the second actuating chamber 121c, and a power unit 25 configured to drive the camshaft 24 into rotation. Each of an end of the plunger assembly 21, an end of the first valve actuator assembly 22 and an end of the second valve actuator assembly 23 abuts against a rotating surface of the camshaft 24 and is slidable on the rotating surface of the camshaft 24. During rotation, the camshaft 24 drives the plunger assembly 21, the first valve actuator assembly 22, and the second valve actuator assembly 23 to reciprocate in timing sequence to squeeze the elastic membrane, in order to generate a directional flow of the liquid in the flow channel 1a.

In the infusion cassette according to the embodiment of the disclosure, each of the plurality of components of the rigid assembly 10 is a rigid injection molding part, and the elastic membrane is a flexible injection molding part. The injection molding part has high processing accuracy, simple assembly, small assembly error and good product consistency. In addition, since the elastic membrane is arranged between two adjacent components of the plurality of components, when the elastic membrane is squeezed by the plunger assembly 21, the first valve actuator assembly 22 and the second valve actuator assembly 23, the possibility of unexpected displacement and swing of the elastic membrane is very small. Furthermore, each of the plunger assembly 21, the first valve actuator assembly 22 and the second valve actuator assembly 23 is provided with an end which is configured to press the elastic membrane and which may be formed into an arc surface. The shape of the arc surface of the plunger assembly is adapted to the shape of the pump chamber 121a, the shape of the arc surface of the first valve actuator assembly is adapted to the shape of the first actuating chamber 121b, and the shape of the arc surface of the second valve actuator assembly is adapted to the shape of the second actuating chamber 121c. Upon the press, the shape of the end of the plunger assembly 21 and the shape of the pump chamber 121a may be adapted to each other, the shape of the end of the first valve actuator assembly 22 and the shape of the first actuating chamber 121b may be adapted to each other, and the shape of the end of the second valve actuator assembly 23 and the shape of the second actuating chamber 121c may be adapted to each other. In this way, the gap processing requirements for mounting holes configured to accommodate the plunger assembly 21, the first valve actuator assembly 22 and the second valve actuator assembly 23 respectively on the pump chassis 28 are reduced, and the liquid stop effect is good, which may relax restrictions on the manufacturing tolerance of the liquid stop plug 162. In addition, the elastic membrane itself has good extrusion resistance, and each of the end surface of the plunger 21, the end surface of the first valve actuator assembly 22 and the end surface of the second valve actuator assembly 23 is an arc surface, so that the risk of leakage caused by the damage of the elastic membrane due to repeated pressing is very low.

The infusion pump of the embodiment of the disclosure can be widely applied to various scenes such as average fluid infusion, analgesia, blood transfusion, chemotherapy and the like.

In an embodiment, the first actuating chamber 121b, the pump chamber 121a, and the second actuating chamber 121c are arranged sequentially along a flow direction of the liquid, and spaced apart from each other.

The number of the plurality of components may be two or more. There may be one elastic membrane or a plurality of elastic membranes. In an embodiment, the plurality of components includes a first component 11, a second component 12 and a third component 13, the groove includes a first groove 121 and a second groove 122, and the elastic membrane includes a first elastic membrane 14 and a second elastic membrane 15. The second component 12 is arranged between the first component 11 and the third component 13, and the first groove 121 is formed on a side of the second component 12 facing toward the first component 11. Specifically, part of the structure of the second component 12 is recessed to form the first groove 121 which is open toward the first component 11. The first elastic membrane 14 is arranged between the first component 11 and the second component 12 to cover the first groove 121 in a sealing manner. With reference to FIG. 5, the first elastic membrane 14 and the first groove 121 cooperate with each other to form a part 1a' of the flow channel 1a.

The second groove 122 is formed on a side of the second component 12 away from the first component 11. Another part of the structure of the second component 12 is recessed to form the second groove 122 which is open toward the third component 13. The second groove 122 communicates with the first groove 121 and is located downstream of the first groove 121. That is, the flow channel 1a penetrates from a first side of the second component 12 to a second side of the second component 12, and the liquid flows from a first side of the second component 12 to a second side of the second component 12. The second elastic membrane 15 is arranged between the second component 12 and the third component 13 to cover the second groove 122 in a sealing manner. With reference to FIG. 5, the second elastic membrane 15 and the second groove 122 cooperate with each other to form another part 1a" of the flow channel 1a. A liquid stop plug 162 is arranged on a side of the third component 13 away from the second component 12 and is configured to press the second elastic membrane 15 to close the another part 1a" of the flow channel 1a.

Specifically, with reference to FIG. 2, the first component 11 is provided with a first hole 11a corresponding to the pump chamber 121a, a second hole 11b corresponding to the first actuating chamber 121b, and a third hole 11c corresponding to the second actuating chamber 121c. After the plunger assembly 21 passes through the first hole 11a, the first elastic membrane 14 may be extruded by the plunger assembly 21. After the first valve actuator assembly 22 passes through the second hole 11b, the first elastic membrane 14 may be pressed by the first valve actuator assembly 22. After the second valve actuator assembly 23 passes through the third hole 11c, the first elastic membrane 14 may be pressed by the second valve actuator assembly 23.

Specifically, the third component 13 is provided with a non-blocking hole 13a, and the liquid stop plug 162 passes through the non-blocking hole 13a from the side of the third component 13 away from the second component 12, to squeeze the second elastic membrane 15 against the second groove 122 of the second component 12, so that the liquid cannot flow in the another part 1a" of the flow channel 1a, that is, the flow channel 1a is closed. That is to say, the locking mechanism 16 closes or opens the flow channel 1a by means of the liquid stop plug 162.

In an embodiment, with reference to FIG. 5, a flow passage hole 122a is formed at a trailing end of the second groove 122, and the liquid stop plug 162 presses the second elastic membrane 15 to the periphery of the flow passage hole 122a, to close the flow channel 1a.

In the infusion cassette 1 according to the embodiment of the disclosure, the liquid stop plug 162 is arranged on the side of the third component 13 away from the second component 12, which prevents the locking mechanism 16 from interfering with the second valve actuator assembly 23. Specifically, it is assumed that the liquid stop plug 162 is arranged on a side of the first component 11 facing toward the pump body 2. In this case, the locking mechanism 16 is arranged close to the second valve actuator assembly for the sake of compactness, but the locking mechanism and the second valve actuator assembly are too close and will interfere with each other. Alternatively, in order to avoid interference, the distance between the locking mechanism and the second valve actuator assembly is appropriately increased, so that the length of the infusion cassette 1 and the length of the pump body 2 must be extended, which will lead to the increase of the dimension of the infusion pump. If the infusion pump is used as an ordinary infusion pump, the large dimension of the infusion pump is acceptable to users, but when the infusion pump is used as an ambulatory patient-controlled analgesia pump, the dimension of the infusion pump should be as compact as possible.

In an embodiment, with reference to FIG. 1, the liquid inlet 1b is arranged at a first end of the infusion cassette 1 along the length direction, and the liquid outlet 1c is arranged at a second end of the infusion cassette 1 along the length direction. In a projection on a plane perpendicular to the thickness direction of the infusion cassette 1, the flow channel 1a, the liquid inlet 1b and the liquid outlet 1c are arranged in a straight line. In this way, the infusion cassette 1 itself can be substantially strip-shaped, which can also facilitate the arrangement of the pump body 2 a corresponding structure. It is to be understood that in other embodiments, the flow channel 1a, the liquid inlet 1b and the liquid outlet 1c may not be arranged in a straight line.

In an embodiment, the pump chamber 121a, the first actuating chamber 121b, and the second actuating chamber 121c are arranged in a straight line in a projection on a plane perpendicular to the thickness direction of the infusion cassette 1.

The plunger assembly 21, the first valve actuator assembly 22, and the second valve actuator assembly 23 perform the press in the following order.

S1 stage: when the first valve actuator assembly 22 presses the first elastic membrane 14 to allow the first elastic membrane 14 to abut against a wall surface of the first actuating chamber 121b, the liquid flow at the first actuating chamber 121b is blocked, the second valve actuator assembly 23 is opened, and the plunger assembly 21 presses the first elastic membrane 14 corresponding to the pump chamber 121a to compress the space in the pump chamber 121a, so that the liquid in the pump chamber 121a flows downstream. That is to say, when the plunger assembly 21 presses the first elastic membrane, the second valve actuator assembly 23 located downstream is in an open state.

S2 stage: when the second valve actuator assembly 23 presses the first elastic membrane 14 to allow the first elastic membrane 14 to abut against a wall surface of the second actuating chamber 121c, the liquid flow at the second actuating chamber 121c is blocked, the first valve actuator assembly 22 and the plunger assembly 21 are opened. Accordingly, the region of the first elastic membrane 14 corresponding to the pump chamber 121a returns to the unpressed state, to generate a negative pressure in the pump chamber 121a, so that upstream liquid enters the pump chamber 121a to fill the space in the pump chamber 121a.

The S1 stage and the S2 stage described above are repeatedly circulated to generate a directional flow of the liquid in the flow channel 1a.

In the related art, all peristaltic fingers will fully press the flexible infusion tube rigidly to allow an inner lumen of the tube to be opened or closed. If the pressed part is a flexible PVC tube, the pressed part may has a risk of damage and leak due to a little longer working time of the infusion pump. In addition, the way of rigidly pressing flexible infusion tube by means of the peristaltic fingers is only suitable for average fluid infusion, but not suitable for blood transfusion, since rigid press can lead to necrosis of a large number of blood cells in the infusion tube.

Therefore, in an embodiment of the disclosure, the first valve actuator assembly 22 and the second valve actuator assembly 23 press the first elastic membrane 14 in a full press manner. The plunger assembly 21 presses the first elastic membrane 14 in a half press manner.

It should be noted that taking the first valve actuator assembly 22 as an example, the full press means that the end of the first valve actuator assembly 22 squeeze the first elastic membrane 14 against the wall surface of the first actuating chamber 121b, so that the liquid flow in the first actuating chamber 121b is blocked.

The half press means that the end of the plunger assembly 21 does not squeeze the first elastic membrane 14 against the wall surface of the pump chamber 121a, so that a gap is formed between the first elastic membrane 14 and the wall surface of the pump chamber 121a. The specific dimension of the gap is not limited, as long as the gap allows the liquid to flow between the first elastic membrane 14 and the wall surface of the pump chamber 12a. That is to say, the plunger assembly 21 does not block the liquid flow at the pump chamber 121a.

In this embodiment, since the plunger assembly 21 presses the first elastic membrane 14 in a half press manner, and when the plunger assembly 21 presses the first elastic membrane 14, the second valve actuator assembly 23 located downstream is in an open state, blood cells in blood have little risk of injury from the plunger assembly 21, and the risk of blood cell injury is within the acceptable range of medical clinic. Therefore, the infusion pump of the embodiment of the disclosure can be used for blood transfusion, other average fluid infusion, analgesia, chemotherapy or the like.

Specifically, when the infusion cassette 1 and the pump body 2 are assembled together, the pump body 2 may drive the locking mechanism 16 to switch from the closed state to the open state and may keep the locking mechanism in the open state. When the infusion cassette 1 is disengaged from the pump body 2, the locking mechanism 16 may be automatically switched from the open state to the closed state. That is to say, when the infusion cassette 1 and the pump body 2 are assembled together, the default state of the infusion cassette 1 is a state in which the flow channel 1a is opened. At this time, the liquid in the infusion tube 3 is automatically controlled by the plunger assembly 21, the first valve actuator assembly 22 and the second valve actuator assembly 23 of the pump body 2 synergetically. That is, the locking mechanism 16 is in the open state. When the infusion cassette 1 is disengaged from the pump body 2, the default state of the infusion cassette 1 is a state in which the flow channel 1a is closed. That is, the locking mechanism 16 is in a closed state to prevent liquid free flowing in the infusion cassette 1.

The material of the first elastic membrane 14 must meet the biocompatibility requirements specified in the relevant standards, and have the expected elasticity and extrusion resistance, for example, silica gel. Similarly, the material of the second elastic membrane 15 must meet the biocompatibility requirements specified in the relevant standards, and have the expected elasticity and extrusion resistance, for example, silica gel.

The material of the first elastic membrane 14 may be the same as or different from the material of the second elastic membrane 15, which are not limited herein.

In an embodiment, with reference to FIG. 2, the locking mechanism 16 further includes an elastic member 17, and the elastic member 17 is configured to exert a force on the mounting frame 161, to allow the locking mechanism 16 to keep in the closed state in which the flow channel 1a is closed. That is to say, in the absence of external force, the default state of the locking mechanism 16 is the closed state. When no external force acts on the locking mechanism 16, the locking mechanism 16 is relatively stably in the closed state under the action of the elastic member 17.

The specific structure type of the elastic member 17 is not limited, for example, a tension spring, a compression spring, a torsion spring and other elastomers. In the embodiment of the disclosure, the elastic member 17 is a torsion spring.

In an embodiment, the open state of the locking mechanism 16 includes a first open state, and the locking mechanism 16 keeps in the first open state under the action of an external force. When the external force is absent or the elastic force of the elastic member 17 can overcome the acting force of the external force on the locking mechanism 16, the elastic member 17 can drive the locking mechanism 16 to automatically switch from the first open state to the closed state.

Specifically, in the process of assembling the pump body 2 with the infusion cassette 1, the acting force of the pump chassis 28 of the pump body 2 on the locking mechanism 16 is the external force described above. When the acting force of the pump chassis 28 of the pump body 2 on the locking mechanism 16 overcomes the acting force of the elastic member 17 on the locking mechanism 16, the pump chassis 28 of the pump body 2 drives the locking mechanism 16 to switch from the closed state to the first open state and keeps the locking mechanism 16 in the open state. When the pump chassis 28 of the pump body 2 is disengaged from the infusion cassette 1, the acting force of the head frame 28 of the pump body 2 on the locking mechanism 16 is absent, that is, the external force acting on the locking mechanism 16 is absent, and the elastic member 17 drives the locking mechanism 16 to automatically switch from the first open state to the closed state. That is to say, the locking mechanism 16 is kept in the first open state only when an external force acts on the locking mechanism 16.

That is to say, when the infusion cassette is in snap-fit with the pump body according to the embodiment of the disclosure, the flow channel in the infusion cassette is automatically opened without other operations, which greatly facilitates the users. When the infusion cassette is disengaged from the pump body, the locking mechanism 16 automatically closes the flow channel, which can prevent excessive transfusion or environmental hazards by the liquid medicine due to the free flow of the liquid in the infusion cassette.

In an embodiment, the open state further includes a second open state. With reference to FIG. 4, the locking mechanism 16 includes a latch structure 163 arranged on the mounting frame 161, and the latch structure 163 can be lock fitted with the rigid assembly 10 to lock the locking mechanism 16 in the second open state. It should be noted that in each of the first open state and the second open state, the flow channel 1a is an open state, and the liquid can continuously flow in the flow channel 1a. For example, the air in the infusion set is discharged before infusion in the second open state.

In the case that the pump body 2 is disengaged from the infusion cassette 1, when it is necessary to keep the flow channel 1a in the open state, the latch structure 163 may be lock fitted with the rigid assembly 10. At this time, there is no relative movement between the locking mechanism 16 and the rigid assembly 10, and the locking mechanism 16 is locked in current second open state.

In an embodiment, with reference to FIG. 2, the rigid assembly 10 is provided with two through holes 1d penetrating through the rigid assembly 10 along the thickness direction of the rigid assembly 10. The flow channel 1a is located between the two through holes 1d. The mounting frame 161 includes a cross bar 1612, a connecting bar 1611 and slidable bars 1613. Each of the slidable bars 1613 is arranged in respective one of the two through holes 1d. The connecting bar 1611 is arranged on one side of the rigid assembly 10 along the thickness direction, and the cross bar 1612 is arranged on another side of the rigid assembly 10 opposite to said one side along the thickness direction. A first end of each of the two slidable bars 1613 is connected to the connecting bar 1611, and a second end of each of the two slidable bars 1613 is connected to the cross bar 1612. The liquid stop plug 162 is arranged on the cross bar 1612. When the pump chassis 28 of the pump body 2 and the infusion cassette 1 are assembled together, the head frame 28 of the pump body 2 pushes the connecting bar 1611 to move toward the third component 13, to drive the whole locking mechanism 16 to move synchronously with the movement of the connecting bar 1611. During the movement, the liquid stop plug 162 is gradually disengaged from the second elastic membrane 15.

It should be noted that the cross bar 1612 is separable from the connecting bar 1611, that is, the cross bar 1612 is not integral with the connecting bar 1611. In this way, it is easy for the slidable bars 1613 to pass through the through holes 1d. Specifically, the connecting bar 1611 may be integral with the two slidable bars 1613, for example, an integral injection molding part. Alternatively, the cross bar 1612 may also be integral with the two slidable bars 1613, for example, an integral injection molding part.

In an example of the embodiment of the disclosure, the connecting bar 1611 is integral with the two slidable bars1613.

In order to facilitate the connection between the slidable bars 1613 and the cross bar 1612, in an embodiment, with reference to FIG. 2, each of the two ends of the cross bar 1612 opposite to each other is provided with a connection hole 1612a, which may be a blind hole or a through hole, and is not limited herein. The second end of each of the slidable bars 1613 is inserted through and fixedly arranged in a respective one of the connecting holes 1612a. For example, the slidable bars 1613 is inserted through the connecting holes 1612a and then is bonded or ultrasonic welded to inner walls of the connecting holes 1612a. Alternatively, the slidable bars 1613 are in interference fit with the connecting holes 1612a to fixedly connect the slidable bars 1613 with the connecting holes 1612a by friction.

In order to facilitate rapid positioning of the cross bar 1612 during the assembling, in an embodiment, a circumferential surface of the second end of each of the slidable bars 1613 is provided with a step surface 1613a, and the cross bar 1612 abuts against the step surfaces 1613a. During the assembling, the connecting holes 1612a on the cross bar 1612 are aligned with the slidable bars 1613, the cross bar 1612 is pushed towards the slidable bars 1613 until the cross bar 1612 abuts against the step surfaces 1613a, and then the cross bar 1612 is bonded to the slidable bars 1613. By means of the step surfaces 1613a, the cross bar 1612 and the slidable bars 1613 can be quickly assembled together, and the relative position of the cross bar 1612 and the slidable bars 1613 can be ensured, which can improve the batch consistency of the products.

In an embodiment, the latch structure 163 protrudes from surfaces of the slidable bars 1613, and is located on a side of one of the slidable bars 1613 away from the other of the slidable bars 1613. It can be understood that the latch structure 163 may be arranged on one of the slidable bars 1613, or each of the slidable bars 1613 may be provided with the latch structure 163. The embodiments of the disclosure are described with the latch structures 163 arranged on the two slide bars 1613 as an example.

The specific configuration of the latch structures 163 is not limited herein. For example, in the embodiment of the disclosure, each of the latch structures 163 is a substantially plate structure extending along the length direction of the slidable bars 1613.

With reference to FIG. 2, in an embodiment, chutes 1f are formed on the wall surfaces of the corresponding through holes 1d, and the latch structures 163 are in a rectilinear sliding fit with the chutes 1f along the thickness direction of the infusion cassette 1. That is to say, the latch structures 163 are slidable in the chutes 1f in a reciprocating movement along the thickness direction of the infusion cassette 1. The first component 11 is provided with stoppers 110 protruding towards the through holes. When the slidable bars 1613 slide to a position where the interference between the latch structures 163 and the stoppers 110 is absent, the locking mechanism 16 is capable to swing along the length direction of the infusion cassette, and the latch structures 163 slide into the rims of the chutes 1f and abut against sides of the stoppers 110 facing toward the second component 12, to allow the locking mechanism 16 to be locked in the second open state.

It should be noted that when the locking mechanism 16 is in the first open state, the locking mechanism 16 may be switched to the second open state or the locking mechanism 16 may be switched to the closed state.

In an embodiment, with reference to FIG. 6, a part of the structure of the mounting frame 161 protrudes from the side of the first component 11 facing toward the pump body 2. A protruding post 281 configured to push the mounting frame 161 is formed on a side of the pump chassis 28 facing toward the infusion cassette 1, and an inclined surface 281a is formed on an end of the protruding post 281 facing toward the infusion cassette 1. During the assembling of the infusion cassette 1 with the pump body 2, the inclined surface 281a pushes the mounting frame 161 to move toward the third component 13, to fix the locking mechanism 16 in the first open state.

The inclined surface 281a is configured to position the mounting frame 161. When the infusion cassette 1 and the pump body 2 are assembled together, the inclined surface 281a abuts against the mounting frame 161 to prevent the mounting frame 161 from moving along the thickness direction of the infusion cassette 1 and from swinging along the length direction of the infusion cassette 1. That is to say, the locking mechanism 16 is stationary at the determinated position and irremovable, thus improving the operational reliability of the locking mechanism 16.

In an embodiment, in the projection perpendicular to the thickness direction of the infusion cassette 1, the flow channel 1a extends along a straight line, thus facilitating the structural arrangement of the pump body 2.

In an embodiment of the disclosure, the plunger assembly 21, the first valve actuator assembly 22 and the second valve actuator assembly 23 are driven by the same camshaft 24. On the one hand, the driving structure can be simplified, so that the product structure is compact and portable; and on the other hand, the cost can be reduced.

It should be noted that the plunger assembly 21 may be a single cylinder, or may be a structure composed of multiple components. Similarly, the first valve actuator assembly 22 may be a single cylinder, or may be a structure composed of multiple components. The second valve actuator assembly 23 may be a single cylinder, or may be a structure composed of multiple components. This is not limited in the embodiment of the disclosure.

In an embodiment, with reference to FIG. 2 and FIG. 7, the first groove 121 includes two pressure monitoring chambers 121d, one of the two pressure monitoring chambers 121d is arranged upstream of the first actuating chamber 121b, and the other of the two pressure monitoring chambers 121d is arranged downstream of the second actuating chamber 121c. The first component 11 is provided with fourth holes 11d corresponding to the pressure monitoring chambers 121d. The pump body 2 includes pressure monitoring devices 26, and the pressure monitoring devices 26 abut against the first elastic membrane 14 through the fourth holes 11d. The pressure monitoring devices 26 are configured to monitor a pressure of the liquid in the flow channel 1a and to identify whether the pressure anomaly, occlusion or empty occurs in the flow channel 1a according to a value of the pressure.

In an embodiment, the pump body 2 further includes an ultrasonic monitoring device 27 to monitor the presence of bubbles in the liquid of the flow channel 1a. There may be one ultrasonic monitoring device 27 or a plurality of ultrasonic monitoring devices 27, which is not limited here.

The specific structure of the power unit 25 is not limited. In an embodiment of the disclosure, the power unit 25 includes a motor 251 and a transmission mechanism 252, and the transmission mechanism 252 is configured to connect a rotating shaft of the motor 251 with the camshaft 24. The transmission mechanism 252 may be a chain, a belt, or a transmission engaged gear set. In the embodiment of the disclosure, the transmission mechanism 252 is a gear set including at least two gears. One of the gears is coaxially arranged with the camshaft 24, and the other of the gears is coaxially arranged with the rotating shaft. The two gears may be directly engaged with each other or may be indirectly connected with each other through other intermediate gears.

The transmission mechanism 252 may be a one-stage transmission or a multistage transmission or the like, which is not limited here.

In an embodiment, the motor 251 is a direct current motor, so that the volume of the motor 251 is small. In the embodiment of the disclosure, the camshaft 24 only needs to drive the plunger assembly 21, the first valve actuator assembly 22, and the second valve actuator assembly 23 to reciprocate in timing sequence. Therefore, the torque output of the direct current motor with a small dimension is sufficient to meet the torque required by the camshaft 24. Therefore, the infusion pump in the embodiment of the disclosure may be provided with only one motor. The infusion pump is small in dimension and portable, and is suitable for use as an ambulatory patient-controlled analgesic pump.

In an embodiment, the camshaft 24 is rotatable in a forward direction and a reverse direction. When the camshaft 24 rotates in the forward direction, the camshaft 24 drives the plunger assembly 21, the first valve actuator assembly 22, and the second valve actuator assembly 23 to reciprocate in forward timing sequence, to drive the liquid in the flow channel to flow in the forward direction and enter into the body of the patient. When the camshaft 24 rotates in the reverse direction, the camshaft 24 drives the plunger assembly 21, the first valve actuator assembly 22, and the second valve actuator assembly 23 to reciprocate in reverse timing sequence, to drive the liquid in the flow channel to flow in the reverse direction. When the occlusion happens during delivery, the camshaft 24 can be controlled to rotate in the reverse direction accurately, so that the infusion pump draws the liquid back, which may release the occlusion pressure and greatly reduce the risk of harm to the patient caused by the occlusion pressure.

The various embodiments/implementation provided in the disclosure may be combined with each other without conflict.

The above descriptions are only preferred embodiments of the disclosure, and are not intended to limit the disclosure. Various modifications and variations of the disclosure are possible for those skilled in the art. Any modification, equivalent replacement, improvement or the like made within the scope of the appended claims shall be included in the scope of protection of the disclosure.

## Claims

1. An infusion cassette (1) of an infusion set, wherein the infusion cassette (1) is provided with a flow channel (1a), and comprises:
a rigid assembly (10) comprising a plurality of components (11, 12, 13) stacked onto one another, wherein at least one of the plurality of components (11, 12, 13) is provided with a groove (121, 122);
an elastic membrane (14, 15) arranged between two adjacent components of the plurality of components (11, 12, 13) to cover the groove (121, 122) in a sealing manner, wherein the elastic membrane (14, 15) and the groove (121, 122) together form at least a portion of the flow channel (1a); and
a locking mechanism (16), wherein the locking mechanism (16) comprises a mounting frame (161) and a liquid stop plug (162) fixedly arranged on the mounting frame (161), the liquid stop plug (162) is configured to press the elastic membrane (14, 15) to close the flow channel (1a), the locking mechanism (16) closes or opens the flow channel (1a) by means of the liquid stop plug (162), wherein the mounting frame (161) is movably connected with the rigid assembly (10) to allow the locking mechanism (16) to switch between a closed state in which the flow channel (1a) is closed and an open state in which the flow channel (1a) is opened,
**characterized in that** the plurality of components (11, 12, 13) comprises a first component (11), a second component (12) and a third component (13); the groove (121, 122) comprises a first groove (121) and a second groove (122); the elastic membrane (14, 15) comprises a first elastic membrane (14) and a second elastic membrane (15); the second component (12) is arranged between the first component (11) and the third component (13); the first groove (121) is formed on a side of the second component (12) facing toward the first component (11), and the first elastic membrane (14) is arranged between the first component (11) and the second component (12) to cover the first groove (121) in a sealing manner; the second groove (122) is formed on a side of the second component (12) away from the first component (11); the second groove (122) communicates with the first groove (121) and is located downstream of the first groove (121); the second elastic membrane (15) is arranged between the second component (12) and the third component (13) to cover the second groove (122) in a sealing manner; the liquid stop plug (162) is arranged on a side of the third component (13) away from the second component (12), and the liquid stop plug (162) is configured to press the second elastic membrane (15) to close the flow channel (1a).

2. The infusion cassette (1) of the infusion set of claim 1, further comprising an elastic member (17) configured to exert a force on the mounting frame (161) to allow the locking mechanism (16) to keep in the closed state in which the flow channel (1a) is closed.

3. The infusion cassette (1) of the infusion set of claim 2, wherein the open state comprises a first open state, and the locking mechanism (16) keeps in the first open state under an action of an external force; wherein when the external force is absent or an elastic force of the elastic member (17) overcomes an acting force of the external force on the locking mechanism (16), the elastic member (17) drives the locking mechanism (16) to automatically switch from the first open state to the closed state.

4. The infusion cassette (1) of the infusion set of claim 3, wherein the open state comprises a second open state, and the locking mechanism (16) comprises a latch structure (163) arranged on the mounting frame (161), wherein the latch structure (163) is configured to be selectively lock fitted with the rigid assembly (10) to lock the locking mechanism (16) in the second open state.

5. The infusion cassette (1) of the infusion set of claim 4, wherein the rigid assembly (10) is provided with two through holes (1d) penetrating through the rigid assembly (10) along a thickness direction of the rigid assembly (10); the flow channel (1a) is located between the two through holes (1d); the mounting frame (161) comprises a cross bar (1612), a connecting bar (1611) and slidable bars (1613); each of the slidable bars (1613) is arranged in respective one of the two through holes (1d); the connecting bar (1611) is arranged on one side of the rigid assembly (10) along the thickness direction, and the cross bar (1612) is arranged on another side of the rigid assembly (10) opposite to said one side along the thickness direction; a first end of each of the two slidable bars (1613) is connected to the connecting bar (1611), and a second end of each of the two slidable bars (1613) is connected to the cross bar (1612); the liquid stop plug (162) is arranged on the cross bar (1612).

6. The infusion cassette (1) of the infusion set of claim 5, wherein the latch structure (163) protrudes from surfaces of the two slidable bars (1613); a chute (1f) is formed on a wall surface of each of corresponding through holes (1d); the first component (11) is provided with a stopper (110) protruding towards the through holes (1d); when the two slidable bars (1613) slide to a position where an interference between the latch structure (163) and the stopper (110) is absent, the locking mechanism (16) is capable to swing along a length direction of the infusion cassette (1), and the latch structure (163) slides into a rim of the chute (1f) and abuts against a side of the stopper (110) facing toward the second component (12), to allow the locking mechanism (16) to be locked in the second open state.

7. An infusion set, **characterized in that** the infusion set comprises: a plurality of infusion tubes (3) and the infusion cassette (1) of the infusion set according to any one of claims 1 to 6, wherein the infusion cassette (1) is provided with a liquid inlet (1b) and a liquid outlet (1c); one infusion tube (3) of the plurality of infusion tubes (3) is connected to the liquid inlet (1b), and another infusion tube (3) of the plurality of infusion tubes (3) is connected to the liquid outlet (1c); and the plurality of infusion tubes (3) communicate with the flow channel (1a).

8. An infusion pump, **characterized in that** the infusion pump comprises: a pump body (2) and an infusion set, wherein the infusion set comprises a plurality of infusion tubes (3) and the infusion cassette (1) of the infusion set according to claim 1; wherein the infusion cassette (1) is provided with a liquid inlet (1b) and a liquid outlet (1c); one infusion tube (3) of the plurality of infusion tubes (3) is connected to the liquid inlet (1b), and another infusion tube (3) of the plurality of infusion tubes (3) is connected to the liquid outlet (1c); and the plurality of infusion tubes (3) communicate with the flow channel (1a); and the pump body (2) and the infusion cassette (1) detachably cooperate with each other,
wherein the first elastic membrane (14) and the first groove (121) together form at least a portion of the flow channel (1a); the first groove (121) comprises a pump chamber (121a), a first actuating chamber (121b) located upstream of the pump chamber (121a), and a second actuating chamber (121c) located downstream of the pump chamber (121a); the pump chamber (121a), the first actuating chamber (121b) and the second actuating chamber (121c) are arranged in a straight line; and
wherein the pump body (2) is arranged on a side of the first component (11) away from the second component (12); the pump body (2) comprises a pump chassis (28), a camshaft (24) rotatably arranged on the pump chassis (28), a plunger assembly (21) press-fitted with the pump chamber (121a), a first valve actuator assembly (22) press-fitted with the first actuating chamber (121b), a second valve actuator assembly (23) press-fitted with the second actuating chamber (121c), and a power unit (25) configured to drive the camshaft (24) into rotation; each of an end of the plunger assembly (21), an end of the first valve actuator assembly (22) and an end of the second valve actuator assembly (23) abuts against a rotating surface of the camshaft (24) and is slidable on the rotating surface of the camshaft (24); the camshaft (24) drives the plunger assembly (21), the first valve actuator assembly (22), and the second valve actuator assembly (23) to reciprocate in timing sequence to press the first elastic membrane (14) during rotation, to generate a directional flow of liquid in the flow channel (1a).

9. The infusion pump of claim 8, wherein the liquid inlet (1b) is arranged at a first end of the infusion cassette (1) along a length direction of the infusion cassette (1), and the liquid outlet (1c) is arranged at a second end of the infusion cassette (1) along the length direction; and the flow channel (1a), the liquid inlet (1b) and the liquid outlet (1c) are arranged along a straight line in a projection on a plane perpendicular to a thickness direction of the infusion cassette (1).

10. The infusion pump of claim 8, wherein the open state comprises a first open state; wherein during assembling of the infusion cassette (1) and the pump body (2), the pump chassis (28) forces the mounting frame (161) to move toward the third component (13), to drive the locking mechanism (16) to switch from the closed state to the first open state and keep the locking mechanism (16) in the first open state.

11. The infusion pump of claim 10, wherein the infusion cassette (1) comprises an elastic member (17) configured to exert a force on the mounting frame (161) to allow the locking mechanism (16) to keep in the closed state in which the flow channel (1a) is closed.

12. The infusion pump of claim 10, wherein the open state comprises a second open state, and the locking mechanism (16) comprises a latch structure (163) arranged on the mounting frame (161); wherein at least one of the first component (11), the second component (12) and the third component (13) is lock fitted with the latch structure (163) to allow the locking mechanism (16) to switch to the second open state and keep the locking mechanism (16) in the second open state.

13. The infusion pump of claim 10, wherein a part of the mounting frame (161) protrudes from a side of the first component (11) facing toward the pump body (2); a protruding post (281) configured to push the mounting frame (161) is formed on a side of the pump chassis (28) facing toward the infusion cassette (1), and an inclined surface (281a) is formed on an end of the protruding post (281) facing toward the infusion cassette (1); wherein during the assembling of the infusion cassette (1) and the pump body (2), the inclined surface (281a) pushes the mounting frame (161) to move toward the third component (13), to fix the locking mechanism (16) in the first open state.

14. The infusion pump of claim 8, wherein the camshaft (24) is rotatable in a forward direction and a reverse direction; wherein when the camshaft (24) rotates in the forward direction, the camshaft (24) drives the plunger assembly (21), the first valve actuator assembly (22) and the second valve actuator assembly (23) to reciprocate in a forward timing sequence, to drive the liquid in the flow channel (1a) to flow in the forward direction; and wherein when the camshaft (24) rotates in the reverse direction, the camshaft (24) drives the plunger assembly (21), the first valve actuator assembly (22) and the second valve actuator assembly (23) to reciprocate in reverse timing sequence, to drive the liquid in the flow channel (1a) to flow in the reverse direction.

## Patentansprüche

1. Infusionskassette (1) eines Infusionssets, wobei die Infusionskassette (1) mit einem Strömungskanal (1a) versehen ist und Folgendes umfasst:
eine starre Baugruppe (10), die eine Vielzahl von aufeinander gestapelten Komponenten (11, 12, 13) umfasst, wobei mindestens eine der Vielzahl von Komponenten (11, 12, 13) mit einer Nut (121, 122) versehen ist;
eine elastische Membran (14, 15), die zwischen zwei benachbarten Komponenten der Vielzahl von Komponenten (11, 12, 13) angeordnet ist, um die Nut (121, 122) dichtend abzudecken, wobei die elastische Membran (14, 15) und die Nut (121, 122) zusammen mindestens einen Abschnitt des Strömungskanals (1a) bilden; und
einen Verriegelungsmechanismus (16), wobei der Verriegelungsmechanismus (16) einen Montagerahmen (161) und einen Flüssigkeitsverschlussstopfen (162) umfasst, der fest auf dem Montagerahmen (161) angeordnet ist, wobei der Flüssigkeitsverschlussstopfen (162) konfiguriert ist, um die elastische Membran (14, 15) zu drücken, um den Strömungskanal (1a) zu verschließen, wobei der Verriegelungsmechanismus (16) den Strömungskanal (1a) mittels des Flüssigkeitsverschlussstopfens (162) verschließt oder öffnet, wobei der Montagerahmen (161) beweglich mit der starren Baugruppe (10) verbunden ist, um dem Verriegelungsmechanismus (16) zu ermöglichen, zwischen einem geschlossenen Zustand, in dem der Strömungskanal (1a) verschlossen ist, und einem offenen Zustand, in dem der Strömungskanal (1a) geöffnet ist, zu wechseln,
**dadurch gekennzeichnet, dass** die Vielzahl von Komponenten (11, 12, 13) eine erste Komponente (11), eine zweite Komponente (12) und eine dritte Komponente (13) umfasst; die Nut (121, 122) eine erste Nut (121) und eine zweite Nut (122) umfasst; die elastische Membran (14, 15) eine erste elastische Membran (14) und eine zweite elastische Membran (15) umfasst; die zweite Komponente (12) zwischen der ersten Komponente (11) und der dritten Komponente (13) angeordnet ist; die erste Nut (121) auf einer der ersten Komponente (11) zugewandten Seite der zweiten Komponente (12) ausgebildet ist, und die erste elastische Membran (14) zwischen der ersten Komponente (11) und der zweiten Komponente (12) angeordnet ist, um die erste Nut (121) dichtend abzudecken; die zweite Nut (122) auf einer von der ersten Komponente (11) abgewandten Seite der zweiten Komponente (12) ausgebildet ist; die zweite Nut (122) mit der ersten Nut (121) in Verbindung steht und der ersten Nut (121) nachgelagert angeordnet ist; die zweite elastische Membran (15) zwischen der zweiten Komponente (12) und der dritten Komponente (13) angeordnet ist, um die zweite Nut (122) dichtend abzudecken; der Flüssigkeitsverschlussstopfen (162) auf einer von der zweiten Komponente (12) abgewandten Seite der dritten Komponente (13) angeordnet ist, und der Flüssigkeitsverschlussstopfen (162) konfiguriert ist, um die zweite elastische Membran (15) zu drücken, um den Strömungskanal (1a) zu verschließen.

2. Infusionskassette (1) des Infusionssets nach Anspruch 1, ferner umfassend ein elastisches Element (17), das konfiguriert ist, um eine Kraft auf den Montagerahmen (161) auszuüben, um es dem Verriegelungsmechanismus (16) zu ermöglichen, in dem geschlossenen Zustand zu bleiben, in dem der Strömungskanal (1a) verschlossen ist.

3. Infusionskassette (1) des Infusionssets nach Anspruch 2, wobei der offene Zustand einen ersten offenen Zustand umfasst und der Verriegelungsmechanismus (16) unter Einwirkung einer externen Kraft in dem ersten offenen Zustand bleibt; wobei, wenn die externe Kraft fehlt oder eine elastische Kraft des elastischen Elements (17) eine wirkende Kraft der externen Kraft auf den Verriegelungsmechanismus (16) überwindet, das elastische Element (17) den Verriegelungsmechanismus (16) antreibt, um automatisch von dem ersten offenen Zustand in den geschlossenen Zustand zu wechseln.

4. Infusionskassette (1) des Infusionssets nach Anspruch 3, wobei der offene Zustand einen zweiten offenen Zustand umfasst und der Verriegelungsmechanismus (16) eine Riegelstruktur (163) umfasst, die an dem Montagerahmen (161) angeordnet ist, wobei die Riegelstruktur (163) konfiguriert ist, um selektiv mit der starren Baugruppe (10) verriegelt zu werden, um den Verriegelungsmechanismus (16) im zweiten offenen Zustand zu verriegeln.

5. Infusionskassette (1) des Infusionssets nach Anspruch 4, wobei die starre Baugruppe (10) mit zwei Durchgangslöchern (1d) versehen ist, die die starre Baugruppe (10) entlang einer Dickenrichtung der starren Baugruppe (10) durchdringen; der Strömungskanal (1a) sich zwischen den beiden Durchgangslöchern (1d) befindet; der Montagerahmen (161) eine Querstrebe (1612), eine Verbindungsstrebe (1611) und verschiebbaren Streben (1613) umfasst; jeder der verschiebbaren Streben (1613) in jeweils einem der beiden Durchgangslöcher (1d) angeordnet ist; die Verbindungsstrebe (1611) auf einer Seite der starren Baugruppe (10) entlang der Dickenrichtung angeordnet ist, und die Querstrebe (1612) auf einer anderen Seite der starren Baugruppe (10) angeordnet ist, die dieser einen Seite entlang der Dickenrichtung gegenüberliegt; ein erstes Ende jedes der beiden verschiebbaren Streben (1613) mit der Verbindungsstrebe (1611) verbunden ist, und ein zweites Ende jedes der beiden verschiebbaren Streben (1613) mit der Querstrebe (1612) verbunden ist; der Flüssigkeitsabsperrstopfen (162) auf der Querstrebe (1612) angeordnet ist.

6. Infusionskassette (1) des Infusionssets nach Anspruch 5, wobei die Riegelstruktur (163) von den Oberflächen der beiden verschiebbaren Streben (1613) hervorsteht; an einer Wandoberfläche jedes der entsprechenden Durchgangslöcher (1d) eine Rinne (1f) ausgebildet ist; die erste Komponente (11) mit einem in Richtung der Durchgangslöcher (1d) vorstehenden Stopfen (110) versehen ist; wenn die beiden verschiebbaren Streben (1613) in eine Position gleiten, in der keine gegenseitige Behinderung zwischen der Riegelstruktur (163) und dem Stopfen (110) mehr vorliegt, der Verriegelungsmechanismus (16) in der Lage ist, entlang einer Längsrichtung der Infusionskassette (1) zu schwingen, und die Riegelstruktur (163) gleitet in einen Rand der Rinne (1f) und stößt gegen eine Seite des Stopfens (110), die in Richtung der zweiten Komponente (12) weist, um eine Verriegelung des Verriegelungsmechanismus (16) im zweiten offenen Zustand zu ermöglichen.

7. Infusionsset, **dadurch gekennzeichnet, dass** das Infusionsset Folgendes umfasst: eine Vielzahl von Infusionsschläuchen (3) und die Infusionskassette (1) des Infusionssets nach einem der Ansprüche 1 bis 6, wobei die Infusionskassette (1) mit einem Flüssigkeitseinlass (1b) und einem Flüssigkeitsauslass (1c) versehen ist; ein Infusionsschlauch (3) der Vielzahl von Infusionsschläuchen (3) mit dem Flüssigkeitseinlass (1b) verbunden ist, und ein anderer Infusionsschlauch (3) der Vielzahl von Infusionsschläuchen (3) mit dem Flüssigkeitsauslass (1c) verbunden ist; und die Vielzahl der Infusionsschläuche (3) mit dem Strömungskanal (1a) in Verbindung steht.

8. Infusionspumpe, **dadurch gekennzeichnet, dass** die Infusionspumpe Folgendes umfasst: einen Pumpenkörper (2) und ein Infusionsset, wobei das Infusionsset eine Vielzahl von Infusionsschläuchen (3) und die Infusionskassette (1) des Infusionssets nach Anspruch 1 umfasst; wobei die Infusionskassette (1) mit einem Flüssigkeitseinlass (1b) und einem Flüssigkeitsauslass (1c) versehen ist; ein Infusionsschlauch (3) der Vielzahl von Infusionsschläuchen (3) mit dem Flüssigkeitseinlass (1b) verbunden ist, und ein anderer Infusionsschlauch (3) der Vielzahl von Infusionsschläuchen (3) mit dem Flüssigkeitsauslass (1c) verbunden ist; und die Vielzahl der Infusionsschläuche (3) mit dem Strömungskanal (1a) in Verbindung steht; und der Pumpenkörper (2) und die Infusionskassette (1) lösbar miteinander zusammenwirken,
wobei die erste elastische Membran (14) und die erste Nut (121) zusammen mindestens einen Teil des Strömungskanals (1a) bilden; die erste Nut (121) eine Pumpenkammer (121a), eine der Pumpenkammer (121a) vorgelagert angeordnete erste Betätigungskammer (121b) und eine der Pumpenkammer (121a) nachgelagert angeordnete zweite Betätigungskammer (121c) umfasst; die Pumpenkammer (121a), die erste Betätigungskammer (121b) und die zweite Betätigungskammer (121c) in einer geraden Linie angeordnet sind; und
wobei der Pumpenkörper (2) auf einer der zweiten Komponente (12) abgewandten Seite der ersten Komponente (11) angeordnet ist; der Pumpenkörper (2) ein Pumpenchassis (28), eine drehbar auf dem Pumpenchassis (28) angeordnete Nockenwelle (24), eine mit der Pumpenkammer (121a) verpresste Kolbenanordnung (21), eine mit der ersten Betätigungskammer (121b) verpresste erste Ventilbetätigungsanordnung (22), eine mit der zweiten Betätigungskammer (121c) verpresste zweite Ventilbetätigungsanordnung (23) und eine Antriebseinheit (25), die konfiguriert ist, um die Nockenwelle (24) in Drehung zu versetzen, umfasst; jeweils ein Ende der Kolbenanordnung (21), ein Ende der ersten Ventilbetätigungsanordnung (22) und ein Ende der zweiten Ventilbetätigungsanordnung (23) an einer rotierenden Oberfläche der Nockenwelle (24) anstoßen und auf der rotierenden Oberfläche der Nockenwelle (24) verschiebbar sind; die Nockenwelle (24) die Kolbenanordnung (21), die erste Ventilbetätigungsanordnung (22) und die zweite Ventilbetätigungsanordnung (23) antreibt, sodass sie sich in zeitlicher Abfolge hin- und herbewegen, um während der Drehung die erste elastische Membran (14) zu drücken und so einen gerichteten Flüssigkeitsfluss im Strömungskanal (1a) zu erzeugen.

9. Infusionspumpe nach Anspruch 8, wobei der Flüssigkeitseinlass (1b) an einem ersten Ende der Infusionskassette (1) entlang einer Längsrichtung der Infusionskassette (1) angeordnet ist und der Flüssigkeitsauslass (1c) an einem zweiten Ende der Infusionskassette (1) entlang der Längsrichtung angeordnet ist; und der Strömungskanal (1a), der Flüssigkeitseinlass (1b) und der Flüssigkeitsauslass (1c) in einer Projektion auf eine Ebene senkrecht zu einer Dickenrichtung der Infusionskassette (1) entlang einer geraden Linie angeordnet sind.

10. Infusionspumpe nach Anspruch 8, wobei der offene Zustand einen ersten offenen Zustand umfasst; wobei während des Zusammenbaus der Infusionskassette (1) und des Pumpenkörpers (2) das Pumpenchassis (28) den Montagerahmen (161) dazu zwingt, sich in Richtung der dritten Komponente (13) zu bewegen, um den Verriegelungsmechanismus (16) dazu anzutreiben, von dem geschlossenen Zustand in den ersten offenen Zustand zu wechseln und den Verriegelungsmechanismus (16) in dem ersten offenen Zustand zu halten.

11. Infusionspumpe nach Anspruch 10, wobei die Infusionskassette (1) ein elastisches Element (17) umfasst, das konfiguriert ist, um eine Kraft auf den Montagerahmen (161) auszuüben, um es dem Verriegelungsmechanismus (16) zu ermöglichen, in dem geschlossenen Zustand zu bleiben, in dem der Strömungskanal (1a) verschlossen ist.

12. Infusionspumpe nach Anspruch 10, wobei der offene Zustand einen zweiten offenen Zustand umfasst und der Verriegelungsmechanismus (16) eine Riegelstruktur (163) umfasst, die am Montagerahmen (161) angeordnet ist; wobei mindestens eine der ersten Komponente (11), der zweiten Komponente (12) und der dritten Komponente (13) mit der Verriegelungsstruktur (163) verriegelt ist, um es dem Verriegelungsmechanismus (16) zu ermöglichen, in den zweiten offenen Zustand zu wechseln und den Verriegelungsmechanismus (16) im zweiten offenen Zustand zu halten.

13. Infusionspumpe nach Anspruch 10, wobei ein Teil des Montagerahmens (161) von einer dem Pumpenkörper (2) zugewandten Seite der ersten Komponente (11) hervorsteht; ein hervorstehender Pfosten (281), der konfiguriert ist, um den Montagerahmen (161) zu drücken, an einer Seite des Pumpenchassis (28) ausgebildet ist, die der Infusionskassette (1) zugewandt ist, und eine geneigte Oberfläche (281a) an einem Ende des hervorstehenden Pfostens (281) ausgebildet ist, das der Infusionskassette (1) zugewandt ist; wobei während des Zusammenbaus der Infusionskassette (1) und des Pumpenkörpers (2) die geneigte Oberfläche (281a) den Montagerahmen (161) drückt, um sich in Richtung der dritten Komponente (13) zu bewegen, um den Verriegelungsmechanismus (16) im ersten offenen Zustand zu fixieren.

14. Infusionspumpe nach Anspruch 8, wobei die Nockenwelle (24) in eine Vorwärtsrichtung und eine Rückwärtsrichtung drehbar ist; wobei, wenn sich die Nockenwelle (24) in der Vorwärtsrichtung dreht, die Nockenwelle (24) die Kolbenanordnung (21), die erste Ventilbetätigungsanordnung (22) und die zweite Ventilbetätigungsanordnung (23) antreibt, um sie in einer Vorwärts-Zeitsequenz hinund herzubewegen, um die Flüssigkeit in dem Strömungskanal (1a) anzutreiben, in die Vorwärtsrichtung zu fließen; und wobei, wenn sich die Nockenwelle (24) in die Rückwärtsrichtung dreht, die Nockenwelle (24) die Kolbenanordnung (21), die erste Ventilbetätigungsanordnung (22) und die zweite Ventilbetätigungsanordnung (23) antreibt, um sie in einer Rückwärts-Zeitsequenz hin- und herzubewegen, um die Flüssigkeit in dem Strömungskanal (1a) anzutreiben, in die Rückwärtsrichtung zu fließen.

## Revendications

1. Cassette de perfusion (1) d'un ensemble de perfusion, dans laquelle la cassette de perfusion (1) est pourvue d'un canal d'écoulement (1a), et comprend :
un ensemble rigide (10) comprenant une pluralité de composants (11, 12, 13) empilés les uns sur les autres, dans laquelle au moins l'un parmi la pluralité de composants (11, 12, 13) est pourvu d'une rainure (121, 122) ;
une membrane élastique (14, 15) agencée entre deux composants adjacents de la pluralité de composants (11, 12, 13) pour couvrir la rainure (121, 122) d'une manière étanche, dans laquelle la membrane élastique (14, 15) et la rainure (121, 122) forment ensemble au moins une partie du canal d'écoulement (1a) ; et
un mécanisme de verrouillage (16), dans laquelle le mécanisme de verrouillage (16) comprend un cadre de montage (161) et un bouchon d'arrêt de liquide (162) agencé fixement sur le cadre de montage (161), le bouchon d'arrêt de liquide (162) est conçu pour presser la membrane élastique (14, 15) pour fermer le canal d'écoulement (1a), le mécanisme de verrouillage (16) ferme ou ouvre le canal d'écoulement (1a) au moyen du bouchon d'arrêt de liquide (162), dans laquelle le cadre de montage (161) est relié de manière mobile à l'ensemble rigide (10) pour permettre au mécanisme de verrouillage (16) de commuter entre un état fermé dans lequel le canal d'écoulement (1a) est fermé et un état ouvert dans lequel le canal d'écoulement (1a) est ouvert,
**caractérisée en ce que** la pluralité de composants (11, 12, 13) comprend un premier composant (11), un deuxième composant (12) et un troisième composant (13) ; la rainure (121, 122) comprend une première rainure (121) et une seconde rainure (122) ; la membrane élastique (14, 15) comprend une première membrane élastique (14) et une seconde membrane élastique (15) ; le deuxième composant (12) est agencé entre le premier composant (11) et le troisième composant (13) ; la première rainure (121) est formée sur un côté du deuxième composant (12) faisant face vers le premier composant (11), et la première membrane élastique (14) est agencée entre le premier composant (11) et le deuxième composant (12) pour couvrir la première rainure (121) d'une manière étanche ; la seconde rainure (122) est formée sur un côté du deuxième composant (12) à l'écart du premier composant (11) ; la seconde rainure (122) communique avec la première rainure (121) et est localisée en aval de la première rainure (121) ; la seconde membrane élastique (15) est agencée entre le deuxième composant (12) et le troisième composant (13) pour couvrir la seconde rainure (122) d'une manière étanche ; le bouchon d'arrêt de liquide (162) est agencé sur un côté du troisième composant (13) à l'écart du deuxième composant (12), et le bouchon d'arrêt de liquide (162) est conçu pour presser la seconde membrane élastique (15) pour fermer le canal d'écoulement (1a).

2. Cassette de perfusion (1) de l'ensemble de perfusion selon la revendication 1, comprenant en outre un élément élastique (17) conçu pour exercer une force sur le cadre de montage (161) pour permettre au mécanisme de verrouillage (16) de rester dans l'état fermé dans lequel le canal d'écoulement (1a) est fermé.

3. Cassette de perfusion (1) de l'ensemble de perfusion selon la revendication 2, dans laquelle l'état ouvert comprend un premier état ouvert, et le mécanisme de verrouillage (16) reste dans le premier état ouvert sous une action d'une force externe ; dans laquelle lorsque la force externe est absente ou qu'une force élastique de l'élément élastique (17) surmonte une force d'action de la force externe sur le mécanisme de verrouillage (16), l'élément élastique (17) entraîne le mécanisme de verrouillage (16) pour commuter automatiquement du premier état ouvert à l'état fermé.

4. Cassette de perfusion (1) de l'ensemble de perfusion selon la revendication 3, dans laquelle l'état ouvert comprend un second état ouvert, et le mécanisme de verrouillage (16) comprend une structure de loquet (163) agencée sur le cadre de montage (161), dans laquelle la structure de loquet (163) est conçue pour être sélectivement mise en verrouillage avec l'ensemble rigide (10) pour verrouiller le mécanisme de verrouillage (16) dans le second état ouvert.

5. Cassette de perfusion (1) de l'ensemble de perfusion selon la revendication 4, dans laquelle l'ensemble rigide (10) est pourvu de deux trous traversants (1d) pénétrant à travers l'ensemble rigide (10) le long d'une direction en épaisseur de l'ensemble rigide (10) ; le canal d'écoulement (1a) est localisé entre les deux trous traversants (1d) ; le cadre de montage (161) comprend une barre transversale (1612), une barre de liaison (1611) et des barres coulissantes (1613) ; chacune des barres coulissantes (1613) est agencée dans l'un respectif des deux trous traversants (1d) ; la barre de liaison (1611) est agencée sur un côté de l'ensemble rigide (10) le long de la direction en épaisseur, et la barre transversale (1612) est agencée sur un autre côté de l'ensemble rigide (10) opposé audit côté le long de la direction en épaisseur ; une première extrémité de chacune des deux barres coulissantes (1613) est reliée à la barre de liaison (1611), et une seconde extrémité de chacune des deux barres coulissantes (1613) est reliée à la barre transversale (1612) ; le bouchon d'arrêt de liquide (162) est agencé sur la barre transversale (1612).

6. Cassette de perfusion (1) de l'ensemble de perfusion selon la revendication 5, dans laquelle la structure de loquet (163) fait saillie de surfaces des deux barres coulissantes (1613) ; une rigole (1f) est formée sur une surface de paroi de chacun des trous traversants (1d) correspondants ; le premier composant (11) est pourvu d'un arrêt (110) faisant saillie en direction des trous traversants (1d) ; lorsque les deux barres coulissantes (1613) coulissent à une position où une interférence entre la structure de loquet (163) et l'arrêt (110) est absent, le mécanisme de verrouillage (16) est capable de pivoter le long d'une direction de longueur de la cassette de perfusion (1), et la structure de loquet (163) coulisse dans un rebord de la rigole (1f) et vient en butée contre un côté de l'arrêt (110) faisant face vers le deuxième composant (12), pour permettre au mécanisme de verrouillage (16) d'être verrouillé dans le second état ouvert.

7. Ensemble de perfusion, **caractérisé en ce que** l'ensemble de perfusion comprend : une pluralité de tubes de perfusion (3) et la cassette de perfusion (1) de l'ensemble de perfusion selon l'une quelconque des revendications 1 à 6, dans lequel la cassette de perfusion (1) est pourvue d'une entrée de liquide (1b) et d'une sortie de liquide (1c); un tube de perfusion (3) de la pluralité de tubes de perfusion (3) est raccordé à l'entrée de liquide (1b), et un autre tube de perfusion (3) de la pluralité de tubes de perfusion (3) est raccordé à la sortie de liquide (1c) ; et la pluralité de tubes de perfusion (3) communiquent avec le canal d'écoulement (1a).

8. Pompe à perfusion, **caractérisée en ce que** la pompe à perfusion comprend : un corps de pompe (2) et un ensemble de perfusion, dans laquelle l'ensemble de perfusion comprend une pluralité de tubes de perfusion (3) et la cassette de perfusion (1) de l'ensemble de perfusion selon la revendication 1 ; dans laquelle la cassette de perfusion (1) est pourvue d'une entrée de liquide (1b) et d'une sortie de liquide (1c) ; un tube de perfusion (3) de la pluralité de tubes de perfusion (3) est raccordé à l'entrée de liquide (1b), et un autre tube de perfusion (3) de la pluralité de tubes de perfusion (3) est raccordé à la sortie de liquide (1c) ; et la pluralité de tubes de perfusion (3) communiquent avec le canal d'écoulement (1a) ; et le corps de pompe (2) et la cassette de perfusion (1) coopèrent de façon détachable l'un avec l'autre,
dans laquelle la première membrane élastique (14) et la première rainure (121) forment ensemble au moins une partie du canal d'écoulement (1a) ; la première rainure (121) comprend une chambre de pompe (121a), une première chambre d'actionnement (121b) localisée en amont de la chambre de pompe (121a), et une seconde chambre d'actionnement (121c) localisée en aval de la chambre de pompe (121a) ; la chambre de pompe (121a), la première chambre d'actionnement (121b) et la seconde chambre d'actionnement (121c) sont agencées dans une ligne droite ; et
dans laquelle le corps de pompe (2) est agencé sur un côté du premier composant (11) à l'écart du deuxième composant (12) ; le corps de pompe (2) comprend un châssis de pompe (28), un arbre à cames (24) agencé de manière rotative sur le châssis de pompe (28), un ensemble piston (21) monté à ajustement serré avec la chambre de pompe (121a), un premier ensemble actionneur de valve (22) monté à ajustement serré avec la première chambre d'actionnement (121b), un second ensemble actionneur de valve (23) monté à ajustement serré avec la seconde chambre d'actionnement (121c) et une unité de puissance (25) conçue pour entraîner l'arbre à cames (24) en rotation ; chacune parmi une extrémité de l'ensemble piston (21), une extrémité du premier ensemble actionneur de valve (22) et une extrémité du second ensemble actionneur de valve (23) vient en butée contre une surface en rotation de l'arbre à cames (24) et peut coulisser sur la surface en rotation de l'arbre à cames (24) ; l'arbre à cames (24) entraîne l'ensemble piston (21), le premier ensemble actionneur de valve (22) et le second ensemble actionneur de valve (23) dans un mouvement de va-et-vient dans une séquence de synchronisation pour presser la première membrane élastique (14) pendant la rotation, pour générer un écoulement directionnel de liquide dans le canal d'écoulement (1a).

9. Pompe à perfusion selon la revendication 8, dans laquelle l'entrée de liquide (1b) est agencée au niveau d'une première extrémité de la cassette de perfusion (1) le long d'une direction de longueur de la cassette de perfusion (1), et la sortie de liquide (1c) est agencée au niveau d'une seconde extrémité de la cassette de perfusion (1) le long de la direction de longueur ; et le canal d'écoulement (1a), l'entrée de liquide (1b) et la sortie de liquide (1c) sont agencés le long d'une ligne droite dans une projection sur un plan perpendiculaire à une direction d'épaisseur de la cassette de perfusion (1).

10. Pompe à perfusion selon la revendication 8, dans laquelle l'état ouvert comprend un premier état ouvert ; dans laquelle pendant l'assemblage de la cassette de perfusion (1) et du corps de pompe (2), le châssis de pompe (28) force le cadre de montage (161) à se déplacer en direction du troisième composant (13), pour entraîner le mécanisme de verrouillage (16) à commuter de l'état fermé au premier état ouvert et garder le mécanisme de verrouillage (16) dans le premier état ouvert.

11. Pompe à perfusion selon la revendication 10, dans laquelle la cassette de perfusion (1) comprend un élément élastique (17) conçu pour exercer une force sur le cadre de montage (161) pour permettre au mécanisme de verrouillage (16) de rester dans l'état fermé dans lequel le canal d'écoulement (1a) est fermé.

12. Pompe à perfusion selon la revendication 10, dans laquelle l'état ouvert comprend un second état ouvert, et le mécanisme de verrouillage (16) comprend une structure de loquet (163) agencée sur le cadre de montage (161) ; dans laquelle au moins l'un du premier composant (11), du deuxième composant (12) et du troisième composant (13) est mis en verrouillage avec la structure de loquet (163) pour permettre au mécanisme de verrouillage (16) de commuter vers le second état ouvert et garder le mécanisme de verrouillage (16) dans le second état ouvert.

13. Pompe à perfusion selon la revendication 10, dans laquelle une pièce du cadre de montage (161) fait saillie d'un côté du premier composant (11) faisant face vers le corps de pompe (2) ; un tenon en saillie (281) conçu pour pousser le cadre de montage (161) est formé sur un côté du châssis de pompe (28) faisant face vers la cassette de perfusion (1), et une surface inclinée (281a) est formée sur une extrémité du tenon en saillie (281) faisant face vers la cassette de perfusion (1) ; dans laquelle pendant l'assemblage de la cassette de perfusion (1) et du corps de pompe (2), la surface inclinée (281a) pousse le cadre de montage (161) à se déplacer en direction du troisième composant (13), pour placer le mécanisme de verrouillage (16) dans le premier état ouvert.

14. Pompe à perfusion selon la revendication 8, dans laquelle l'arbre à cames (24) est rotatif dans une direction vers l'avant et une direction inverse ; dans laquelle lorsque l'arbre à cames (24) fait une rotation dans la direction vers l'avant, l'arbre à cames (24) entraîne l'ensemble piston (21), le premier ensemble actionneur de valve (22) et le second ensemble actionneur de valve (23) dans un mouvement de va-et-vient dans une séquence de synchronisation vers l'avant, pour entraîner le liquide dans le canal d'écoulement (1a) en écoulement dans la direction vers l'avant ; et dans laquelle lorsque l'arbre à cames (24) fait une rotation dans la direction inverse, l'arbre à cames (24) entraîne l'ensemble piston (21), le premier ensemble actionneur de valve (22) et le second ensemble actionneur de valve (23) dans un mouvement de va-et-vient dans une séquence de synchronisation inverse, pour entraîner le liquide dans le canal d'écoulement (1a) en écoulement dans la direction inverse.
